# EUROPEAN PATENT SPECIFICATION

(11) **EP 1 925 259 B1**
(45) Date of publication and mention of the grant of the patent: **18.01.2012**
(21) Application number: 07254539.5
(22) Date of filing: 21.11.2007
(51) Int. Cl.: A61B 17/32

(54) **Skin-piercing device for treatment of acne**
Vorrichtung zum Durchstechen der Haut bei der Behandlung von Akne
Dispositif percutané pour le traitement de l'acné

(30) Priority: 22.11.2006 US 860703 P; 23.03.2007 US 896536 P
(43) Date of publication of application: 28.05.2008
(73) Proprietor: Johnson and Johnson Consumer Companies, Inc., Skillman, NJ 08558 (US)
(72) Inventor: Wu, Jeffrey M., Princeton, New Jersey 08540 (US); McLaughlin, Robert A., Milltown, New Jersey 08850 (US)
(74) Representative: James, Anthony Christopher W.P.

(56) References cited:
- GB-A- 2 331 935
- US-A- 4 994 068
- US-A- 5 354 307
- US-B1- 6 258 112

## Description

### BACKGROUND OF INVENTION

Acne is a common skin disease characterized by pimples on the face, chest, and back. It occurs when the pores of the skin become clogged with oil, dead skin cells and bacteria. Inflamed lesions may cause pain, tenderness, itching, or swelling. The most troubling aspects of these lesions are negative cosmetic effects.

Pimple popping has been a pervasive, but sometime impulsive, behavior to quickly reduce the elevation of raised acne, especially pustule type of lesions. The current pimple popping practice is to use fingers to squeeze the pimple, which can carry the risks of infections, post-inflammatory hyperpigmentation, and even scarring. While sterilized hypodermal needles can be used to break the tip of the pimple to release the pus, such needles do not control the depth of and size of cut, which can cause unwanted bleeding and skin damages. Thus, there is clearly a need to provide a safe and hygienic way to pop the pimple and reduce the symptoms of pimple.

The present invention discloses a device for carrying out a new method and device to safely assist popping pimple to remove puss and/or treat acne as well as other skin disorders such as cold sores, warts, razor bumps, and age spots.

US-B-6258112 describes a single use blood-sampling lancet assembly comprising a housing having an opening; a lancet disposed within the housing; a forcing assembly urging the lancet in a direction to project the tip of the lancet from within the housing through the opening; a trigger assembly arranged to retain the lancet in a retracted position energizing the forcing assembly and actuable to cause the tip of the lancet to emerge from the opening.

GB-A-2331935 describes a cartridge for sampling blood including a housing containing an extendable blood sampling lancet, the housing having a compartment for receiving the blood which includes an analytical region for analysing a property of the blood.

### SUMMARY OF THE INVENTION

In one aspect, the present invention features a skin-piercing device for the treatment of acne by pimple popping including: a housing having a skin-contacting surface, wherein the housing includes an opening in the skin-contacting surface; a piercing element disposed within the housing; a forcing assembly urging the piercing element in a direction to project the tip of the piercing element from within the housing through the opening in the skin-contacting surface of the housing; a trigger assembly with a portion contained within the housing arranged to retain the piercing element in a retracted position energizing the forcing assembly and a second portion outside the housing manually actuable to release the piercing element to cause the tip to have a momentary position projecting from the opening; and characterized in that a fluid-absorbent material is affixed to the outside of said housing and wherein said skin-piercing device is adapted such that the effective length of the piercing element tip that projects from the skin-piercing device is from 100 micrometers to 500 micrometers.

In a second aspect, the invention features a kit including a skin-piercing device according to the first aspect of the invention, and further comprising at least one of the following additional items: (i) a composition including an anti-acne agent, (ii) a re-cocking element to position the piercing element into the retracted position following the release the piercing element, and (iii) an aiming device to assist in positioning the skin-piercing device over the area of skin to be pierced.

Other aspects, features, and advantages of the present invention will be apparent from the detailed description of the invention and from the claims.

### DETAILED DESCRIPTION OF THE DRAWINGS

FIG. 1 is a side cross-section view of a skin-piercing device of the present invention in an unused, un-cocked orientation.
FIG. 2 is a side cross-section view of a skin-piercing device of the present invention in a cocked orientation.
FIG. 3 is a side cross-section view of a skin-piercing device of the present invention in a piercing orientation
FIG. 4 is a side cross-section view of a skin-piercing device of the present invention in a piercing orientation.
FIG. 5 is a side cross-section view of a portion of a skin-piercing device of the present invention containing a fluid-absorbent material on the skin-contacting surface.
FIG. 6 is a side cross-section view of a portion of a skin-piercing device of the present invention containing a ridge on the skin-contacting surface.
FIG. 7 is a side cross-section view of a skin-piercing device of the present invention that is capable of being repositioned in the cocked orientation.
FIG. 8A is a top view of an aiming device of the present invention.
FIG. 8B is a bottom view of an aiming device of the present invention.
FIG. 9 is a side view of the use of the skin-piercing device and aiming device of the present invention.

### DETAILED DESCRIPTION OF THE INVENTION

It is believed that one skilled in the art can, based upon the description herein, utilize the present invention to its fullest extent. The following specific embodiments can be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. As used herein, all percentages are by weight unless otherwise specified.

In one embodiment, the present invention is directed to a skin-piercing device as defined in claim 1 for treating a skin-condition such as acne. The treatment involves disrupting the stratum corneum of the skin and may or may not further include the application of a composition that permeates into the disrupted skin, such as a composition containing an anti-acne agent. A benefit of such a treatment includes localizing the treatment to a certain area of skin in need of such treatment.

### Definitions

What is meant by a "product" is a product in finished packaged form. In one embodiment, the package is a container such as a plastic, metallic, or cardboard box for storing such skin-piercing device and/or kit. In one embodiment, the product contains instructions directing the user to apply the skin-piercing device to the skin (e.g., for the treatment of a skin condition such as acne).

What is meant by "promoting" is promoting, advertising, or marketing. Examples of promoting include, but are not limited to, written, visual, or verbal statements made on the product or in stores, magazines, newspaper, radio, television, internet, and the like. For promoting the treatment of acne, examples of such statements include, but are not limited to, "treats acne," "safely pops pimples," "eliminates acne and/or pimples/blemishes", and "visibly reduces the symptoms and/or appearance of pimples." Similar statements can be made for other skin disorders.

The term "treating" or "treatment" of a skin disorder means the treatment (e.g., complete or partial alleviation or elimination of symptoms and/or cure) and/or prevention or inhibition of the skin disorder.

As used herein, "composition" means a composition suitable for administration to the skin.

As used herein, "cosmetically-acceptable" means that the ingredients or compositions which the term describes are suitable for use in contact with the skin without undue toxicity, incompatibility, instability, irritation, allergic response, and the like. This term is not intended to limit the ingredient/composition to which it describes for use solely as a cosmetic (e.g., the ingredient/composition may be a pharmaceutical agent).

As used herein, "safe and effective amount" means an amount of the active agent, compound, carrier, or of the composition sufficient to induce the desired effect, but low enough to avoid serious side effects. The safe and effective amount of the compounds or composition will vary with the area being treated, the age, health and skin/tissue type of the end user, the duration and nature of the treatment, the specific compound or composition employed, the particular cosmetically-acceptable carrier utilized, and like factors.

### Skin-piercing device

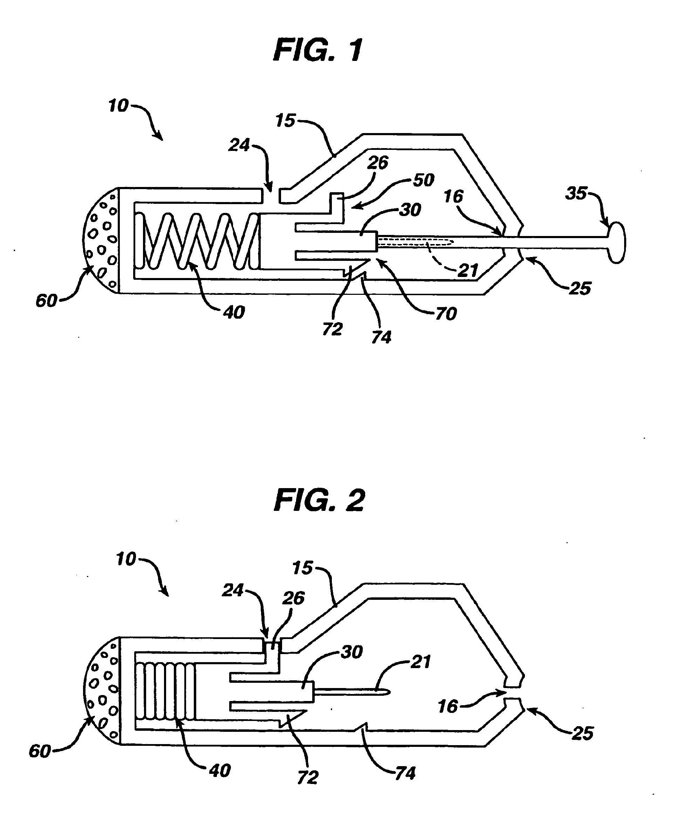

In one embodiment (as depicted in the FIG. 1), the skin-piercing device of the present invention is a single-use, skin-piercing device **10.** Other configurations of single-use lancet devices may also be used, such as those depicted in US Patent Nos. 5,487,748, 6,258,112, and 6,764,496.

The skin-piercing device **10** includes housing **15.** Housing **15** may be made of various rigid materials, including but not limited to plastic and metal. The housing **15** includes two openings, piercing element opening **16** (from which piercing element tip **21** exits the housing **15)** on the skin-contacting surface **25** of housing **15** and trigger opening **24** (from which trigger button **26** can be accessed through housing **15).**

In one embodiment, the skin-contacting surface 25 has a surface area of less than 50 mm², such as from about 0.5 mm² to about 50 mm², such as from about 1 mm² to about 25 mm². Such a small surface area allows the user to feel where the piercing element tip 21 will exit the skin-piercing device, for example, to position the piercing element opening 16 over the pimple.

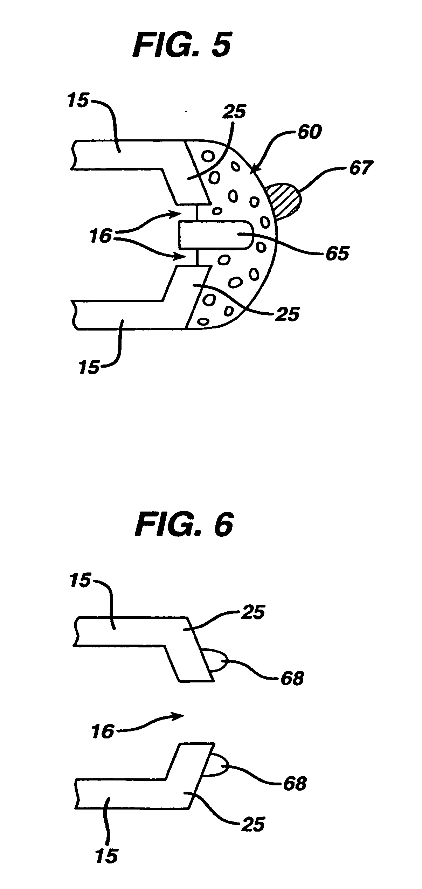

In one embodiment, the skin-contacting surface 25 includes a concave shape and piercing element opening 16 is within this concave shape. Such shape allows the user to place the skin-contacting surface, and consequently the piercing element opening 22, over the pimple. In one embodiment, the skin-contacting 25 includes a circumferential ridge 68 (shown in FIG 6) around piercing element opening 16. Such as ridge can assist the user in positioning the device on the skin surface and/or enhance the vertical pressure on the skin before, during, and after the skin is pierced by the piercing element tip 21, thereby assisting in the removal of the bodily fluids (such as pus from a pimple).

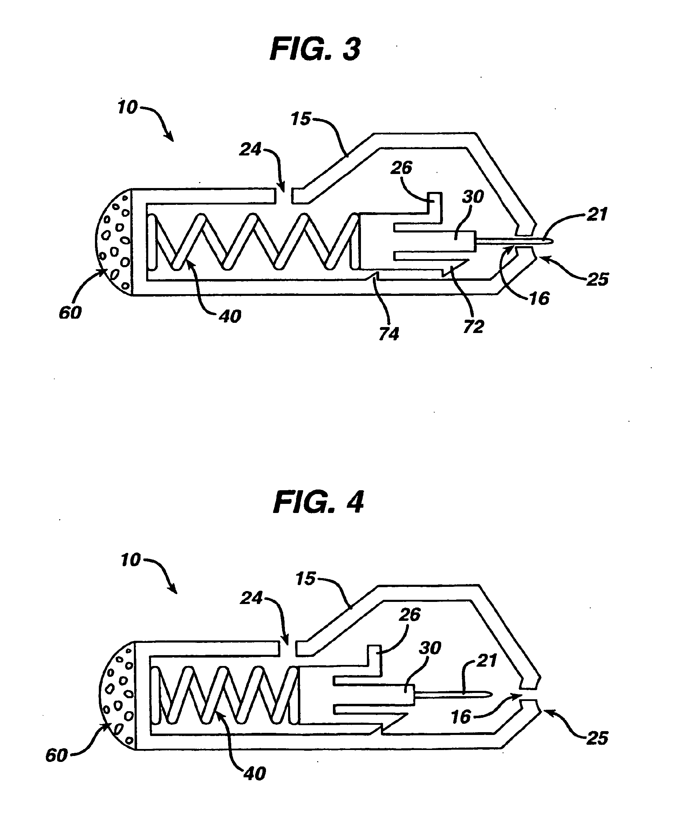

Piercing element 30 includes at least one piercing element tip 21 that pierces the skin following activation of skin-piercing device 10. In one embodiment, piercing element 30 includes from about 2 to about 10 piercing elements tips. Piercing element tip 21 may be made of into shapes, including a needle (either solid or hollow) or a blade. In one embodiment, the piercing element tip is a needle having a diameter no more than 1.45 mm (gauge of at least 15), such as from 0.143 mm to 1.02 mm (gauge 18 to gauge 35), such as from 0.255 mm to 0.723 (gauge 21 to gauge 30). Piercing element tip 21 may be made of rigid materials capable of piercing the skin, such as metal. Piercing element 30 is arranged within housing 20, such that it can move from an unused, un-cocked orientation as depicted in FIG. 1, then to a cocked orientation as depicted in FIG. 2, then to a piercing orientation as depicted in FIG. 3 (during which tip 21 temporarily exits the housing 15 through piercing element opening 16), and then to a used orientation as depicted in FIG 4.

Piercing element 30 preferably moves through housing 15 in a substantially linear direction. In one embodiment, the movement of piercing element 30 through housing 15 is maintained in a linear direction by the inclusion of one or more ridges (not shown) extending from piercing element 30 into one or more linear tracks in the inner wall of housing 15.

Skin-piercing device 10 in preferable obtained by user prior to use in the unused, un-cocked orientation as depicted in FIG. 1, however, it may also be obtained by the user in the cocked orientation. In one embodiment, tip cover 35 extends into the housing 15 through piercing element opening 16 and at least partially covers tip 21. In one embodiment, tip cover 35 serves the purpose of maintaining the sterility of piercing element tip 21.

Tip cover 35 is arranged such that it may be pushed inward into the housing by the user in order to position the piercing element 30 further away from the piercing element opening 16, thereby positioning the piercing element 30 into a cocked orientation as depicted in FIG. 2. Once in this cocked orientation, tip cover 35 may be disengaged from piercing element 30 (thereby uncovering piercing element tip 21) and removed from housing 15.

Skin-piercing device 10 further includes forcing assembly 40 to urge piercing element 30 in a direction to project piercing element tip 21 from within housing 15 through the piercing element opening 16 with sufficient force such that the tip 21 is capable of piercing the skin. Forcing assembly 40 is configured within housing 15 in order to move piercing element 30 from a cocked orientation as depicted in FIG. 2 to a piercing orientation as depicted in FIG. 3 (during which tip 21 temporarily exits the housing 15 through piercing element opening 16). In one embodiment, as depicted in the Figures, the forcing assembly 40 is positioned between housing 15 and the piercing element 30. The forcing assembly 40 may be a spring, as depicted in the Figures. The forcing assembly 40, however, may also be an compressible, elastic material (such a solid silicone material or urethane foam) or a pneumatic or hydraulic assembly.

The skin-piercing device 10 is also preferably arranged such that after the piercing element tip 21 has exited the housing through piercing element opening 16, the piercing element tip 21 tends to be retracted (e.g., by the forcing assembly 40) back into the housing (as depicted in the used orientation of FIG 4). Such orientation assists in preventing the accidental reuse of the piercing element tip 21.

The skin-piercing device 10 is adapted such that when piercing element tip 21 exits the housing 20, the piercing element tip 21 has an effective length of from 100 micrometers to 500 micrometers, such as from 150 micrometers to 500 micrometers, such as from 150 micrometers to 350 micrometers. What is meant be effective length is the length of the piercing element tip adapted to penetrate into the skin (e.g., the length from the piercing element tip that will penetrate the skin upon activating the skin-piercing device).

Skin-piercing device 10 includes a trigger assembly 50 that serves to both retain the piercing element 30 in a retracted position energizing the forcing assembly 40 and later release piercing element 30 from its cocked orientation. A variety of trigger assemblies can be utilized in the skin-piercing device to serve this purpose. In one embodiment, trigger assembly 50 includes trigger button 26, which is secure to piercing element 30 and is structured to temporarily protrude from housing 15 through trigger opening 24, thereby holding the piercing element 30 in the cocked orientation as depicted in FIG 2. Other trigger assemblies may also be used, such as one secured to the housing as described in US Patent No. 5,487,748. In one embodiment (not shown), the trigger assembly is secured to the piercing element and is also used to position the piercing element into the cocked orientation.

The piercing element **30** may be moved by the forcing assembly **40** from the cocked orientation as depicted in FIG 2 to a piercing orientation as depicted in FIG. 3 by pushing the trigger button **26** back through trigger opening **24** and into the housing **15.**

In one embodiment, skin-piercing device **10** further includes restrictor assembly **70,** which has the purpose of inhibiting piercing element **30** from being repositioned in the cocked orientation once it has moved into the used orientation. In one embodiment, restrictor assembly **70** includes piercing element protrusion **72** (secured to piercing element **30)** and housing protrusion **74** (secured to housing **15).** The restrictor assembly **70** is arranged such that piercing element protrusion **72** may pass over housing protrusion **74** when piercing element **30** moves from the cocked orientation as depicted in FIG. 2 to a piercing orientation as depicted in FIG. 3. However, once piercing element **30** has moved from a piercing orientation as depicted in FIG. 3 (during which tip **21** temporarily exits the housing **15** through piercing element opening **16)** to the used orientation as depicted in FIG. 4, housing protrusion **74** inhibits the movement of piercing element **30** into the cocked orientation by contacting piercing element protrusion **72,** as depicted in FIG. 4. Other restrictor assemblies can also be used, such as that disclosed in PCT Patent Application WO02/43591.

As shown in the Figures, piercing element protrusion **72** is structured such that as it moves from the cocked orientation over housing protrusion **74,** the sloped arrangements of piercing element protrusion **72** and housing protrusion **64** permit piercing element protrusion to bend toward piercing element **30,** thereby allowing piercing element protrusion **72** to pass over housing protrusion **74** as piercing element **30** moves from the cocked orientation as depicted in FIG. 2 to a piercing orientation as depicted in FIG. 3. Piercing element protrusion **72** is also structured such that once in the used orientation, the piercing element protrusion **72** and housing protrusion **74** abut each other, thereby inhibiting the piercing element 30 from being reengaged by the user into the cocked orientation.

In one embodiment, the skin-piercing device of the present invention includes fluid-absorbent material **60.** Absorbent material **60** allows the skin-piercing device **10** to collect bodily fluids such as pus from a pimple pierced by piercing element tip **21.** The fluid-absorbent material may be secured to the skin-contacting surface of the skin-piercing device, or to another surface of the skin-piercing device (such as the opposite end of the skin-piercing device as depicted in the Figures).

In one embodiment, the surface of the fluid-absorbent material includes a guide structure 67 having a diameter of from about 0.5 mm to about 3mm. In one embodiment, the structure is harder than the fluid-absorbent material. Such structure assists the user in position the fluid-absorbent material on the pimple to be treated.

In one embodiment, the fluid-absorbent material may also be used to administer a topical fluid composition (e.g., containing an anti-acne agent as discussed below to the skin in need of such treatment. The skin-piercing device may be packaged such that a composition is (i) added to the fluid-absorbent material proximate to use or (ii) contained within the fluid-absorbent material during storage.

What is meant by fluid-absorbent material is a material that is capable of absorbing fluids in an amount of at least 25 percent of its weight. Examples of absorbent, compressible materials include, but are not limited to, woven and nonwoven materials, hydrogels, hydrocolloids, silicone rubbers, celluloses (e.g., cotton and rayon or their derivatives), wool, polyamides (e.g., nylon), and silk.

In one embodiment (depicted in FIG. 5), fluid-absorbent material 60 encases a reservoir 65 that contains a fluid composition that is expelled from the reservoir 65 upon puncture of the fluid-absorbent material 60 by the piercing element tip. In one embodiment, the fluid composition contains an anti-acne active.

### Recockable Skin-piercing Device

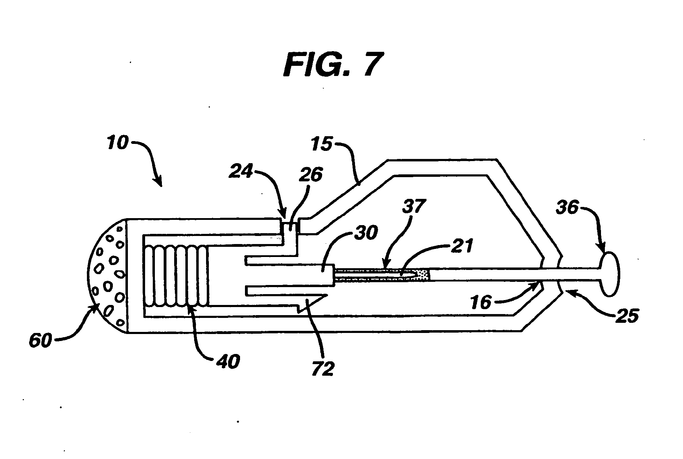

In one embodiment, the piercing element **30** may be repositioned in the cocked orientation once it has moved into the used orientation. As shown in the embodiment of FIG. **7****,** skin-piercing device **10** does not include housing protrusion **74.** Thus, by inserting re-cocking element 36 into skin-piercing device **10,** piercing element **30** may be reposition into the cocked position following use of skin-piercing device **10.** In one embodiment, sterilizing section **37** of re-cocking element **36** engages piercing element **30.** Sterilizing section 37 may comprise an absorbent material (such as a woven material, non-woven material, or porous urethane foam) that further contains a antimicrobial agent, such as hydrogen peroxide, guanidinium thiocyanate, sodium hydroxide, alcohol (such as ethanol or propanol), benzalkonium chloride, benzethonium chloride, methyl benzethonium chloride, lauric argainate, sugarquat, cetylpyridiunium chloride, and 2,4,4'-trichloro-2-hydroxy diphenylether. The antimicrobial agent provides that when sterilizing section 37 contacts piercing element tip **21,** it cleans piercing element tip **21** of any residual blood, pus, or other fluids from its prior use.

### Multiple-Use Skin-piercing device

In one embodiment, the skin-piercing device includes multiple piercing elements. In one embodiment, the skin-piercing device is adapted such that each piercing element may only be used once. Examples of such a skin-piercing device include the ACCU-CHEK® Multiclix Lancet Device (Roche Diagnostics, Basel, Switzerland) and devices set forth U.S. Patent Application No. 2004/0260325. In one embodiment, the device is adapted such that the piercing elements can be replaced after use (e.g., disposable storage case can containing multiple piercing element such as that described in European Patent Application No. 638465).

### Aiming Device

In one embodiment, the user utilizes an aiming device to assist in positioning the skin-piercing device over the area of skin to be pierced (e.g., the pimple). In one embodiment, the aiming device is first positioned over the area of skin, following which the skin-piercing device is then secured to such aiming device such that upon activation, the skin-piercing device pierces that desired area of skin.

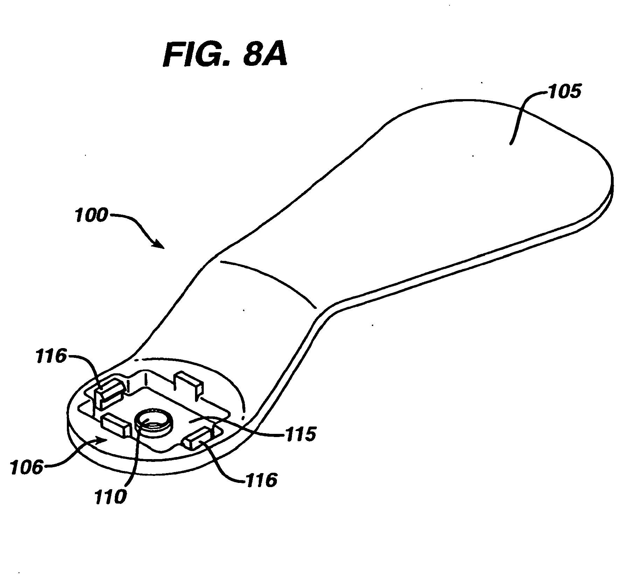

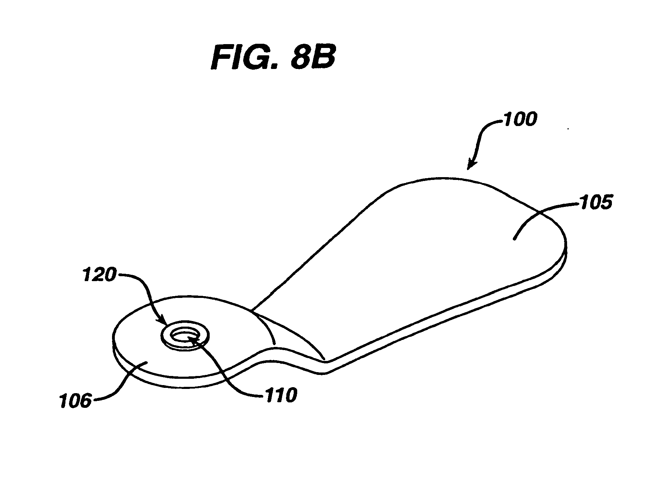

An example of such an aiming device is set forth in FIGS. 8A and 8B. As set forth in FIG. 8A, the aiming device **100** has a handle portion **105** and a skin-contacting portion 106. The top of skin-contacting portion **106,** which is adapted to secure the device to the aiming device **100,** has a device-contacting recess **115** that includes securing tabs **116** and orifice **110.** In one embodiment, the aiming device **100** is made of a transparent material, such as plastic, such that user can determine that the orifice is positioned in the desired location (e.g., over the pimple to be treated).

As shown in FIG. 8b, the bottom of skin-contacting portion **106** is adaped to contact the skin, and includes orifice **110** within ring **120.** By pressing ring **120** against the pimple, the ring **120** applies force on the sides of the pimple, thereby assisting in the removal of pus from the pimple.

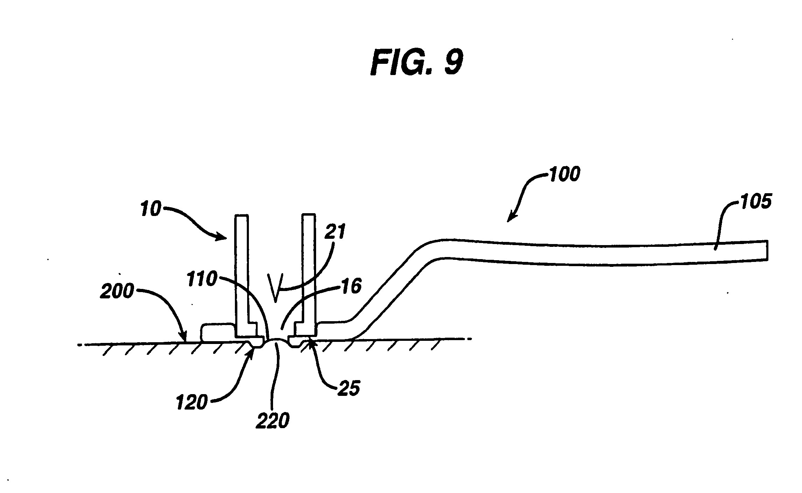

FIG. 9 is a depiction of the aiming device **100** being used in conjunction with skin-piercing device **10.** Aiming device **100** is first pressed on the surface of skin **200** such that ring **120,** and consequently orifice **110,** is positioned over pimple **220.** Skin-contacting surface **25** of skin-piercing device **10** is then positioned within device-contacting recess **115** and secured with securing tabs **116** (not shown) such that the piercing element opening **16** is positioned over orifice **110** and consequently over pimple **220.** Upon activation of skin-piercing device **10,** piercing element tip **21** exits skin-piercing device **10** and enters pimple **220.**

### Topical Compositions

In one embodiment, the skin-piercing device includes, or is used in conjunction with a topical composition. The composition may be solid, semisolid, liquid, or any combination thereof. In particular, examples of solid compositions include but are not limited to bars, sticks, powders, masks, and patches. Examples of semisolid compositions include but are not limited to creams, lotions, gels, ointments, hydrogels, hydrocolloids, foams, mousses, emulsions, and micro-emulsions. Examples of liquid compositions include but are not limited to liquid cleansers, toners, serums, sprays, and aerosols.

In one embodiment, the topical composition includes an anti-acne agent. What is meant by an anti-acne agent is an compound that has been approved by the U.S. Food and Drug Administration for the topical treatment of acne. Examples of anti-acne agents include, but are not limited to, salicylic acid, benzoyl peroxide, sulphur, retinoic acid, isotretinoin, candida bombicola/glucose/methyl rapeseedate ferment, peat water, resorcinol, silt, peat, permethin, azelaic acid, tazorotene, clindamycin, adapalene, erythromycin, sodium sulfacetamide, and combinations thereof. In one embodiment, the amount of anti-acne agent in the composition is from about 0.01 % to about 10%, for example from about 0.1 % to about 5%, or from about 0.5% to about 2% by weight, based on the total weight of the composition.

In one embodiment, the topical composition includes an active agent for the treatment of warts, age spots, razor bumps. Examples of such active agents include ascorbic acid, salicylic acid, and salts and esters thereof.

In one embodiment, the active agent and/or composition is applied to the skin proximate to the time of the piercing of the skin with the skin-piercing device (e.g., within about an hour before or after the piercing, such as within about fifteen minutes or within about five minutes).

### Pre-softening Treatment

In one embodiment, the skin is softened prior to piercing, thereby allowing the skin to be more easily pierced and/or fluids (such as pus) to be more easily removed. Examples of such pre-softening treatments include application of an occlusive or semi-occlusive dressing to the skin piercing site (such as hydrocolloids, hydrogels and transparent film dressings) or hydrating the site (such as cleansing with warm water). Other pre-softening treatments include warming the skin site (e.g., to a temperature from about 35-50°C) or the application of skin softening agents such as enzymes or acids (e.g., hydroxyl acids).

### Kit

In one embodiment, skin-piercing device(s) of the present invention and optionally companion item(s) are packaged together and marketed as a kit. The examples of the items in the kit may include, but are not limited to, one or more of (i) the skin-piercing devices, (ii) topical compositions in a suitable container/dispenser (such as a tube, a bottle, a pump, a jar, a dropper, a or unit-dose dispenser) to be used before, during, or after the stratum-corneum piercing skin-piercing device application, (iii) aiming devices, and/or (iv) re-cocking elements. Additionally, the kit may also contain a cleansing product to be used to sanitize/sterilize the skin prior to the skin-piercing device application. The kit may also include a film forming composition or bandage to be used after treatment to protect the treated skin site, further assist in the removal of pus, and/or to enhance the therapeutic efficacies for the treated skin.

It is understood that while the invention has been described in conjunction with the detailed description thereof, that the foregoing description is intended to illustrate and not limit the scope of the invention, which is defined by the scope of the appended claims.

## Claims

1. A skin-piercing device (10) for the treatment of acne by pimple popping, comprising:
a housing (15) having a skin-contacting surface (25), wherein said housing comprises an opening (16) in said skin-contacting surface,
a piercing element (30) disposed within said housing,
a forcing assembly (40) urging the piercing element in a direction to project the tip (21) of said piercing element (30) from within said housing through said opening (16) in said skin-contacting surface (25) of said housing,
a trigger assembly (50) with a portion contained within said housing arranged to retain said piercing element (30) in a retracted position energizing the forcing assembly (40) and a second portion outside said housing manually actuable to release said piercing element to cause said tip to have a momentary position projecting from said opening, and
**characterized in that** a fluid-absorbent material (60) is affixed to the outside of said housing (15) and wherein said skin-piercing device is adapted such that the effective length of the piercing element tip (21) that projects from the skin-piercing device (10) is from 100 micrometers to 500 micrometers.

2. A skin-piercing device of claim 1, wherein said skin-piercing device (10) further comprises a restrictor assembly (70) structured to substantially prevent said piercing element (30) from moving completely into said retracted position after said tip (21) has projected from said opening (16).

3. A skin-piercing device of any preceding claim, wherein said forcing assembly (40) is a spring.

4. A skin-piercing device of any preceding claim, wherein the surface area of said skin-contacting surface (25) is from 0.5 mm² to 25 mm².

5. A skin-piercing device of any preceding claim, wherein said skin-contacting surface (25) comprises a concave shape and said opening (16) is within said concave shape.

6. A skin-piercing device of any preceding claim, wherein the surface of said fluid-absorbent material (60) comprises a structure having a diameter of from 0.5 mm to 3 mm.

7. A skin-piercing device of any preceding claim, wherein said fluid absorbent material (60) comprises an anti-acne agent.

8. A skin-piercing device of claim 7, wherein said anti-acne agent is selected from the group consisting of salicylic acid and benzoyl peroxide.

9. A skin-piercing device of any preceding claim, wherein said fluid-absorbent material covers said opening and said fluid absorbent material is capable of being pierced by said piercing element tip (21).

10. A kit comprising a skin-piercing device (10) according to any preceding claim, said kit further comprising at least one item selected from the following group of items: (i) a composition comprising an anti-acne agent, (ii) a re-cocking element (36) to position said piercing element (30) into said retracted position following the release of said piercing element, and (iii) an aiming device (100) to assist in positioning the skin-piercing device (10) over the area of skin to be pierced.

11. A kit of claim 10, wherein said kit further comprises at least one of said compositions, wherein said anti-acne agent is selected from the group consisting of salicylic acid, benzoyl peroxide, retinoic acid, azelaic acid, tazorotene, clindamycin, adapalene, erythromycin, sodium sulfacetamide, and combinations thereof.

12. A kit of claim 10 or 11, wherein said kit further comprises at least one of said re-cocking elements (36).

13. A kit of claim 10, 11 or 12, wherein said kit further comprises at least one said aiming devices (100).

## Patentansprüche

1. Hautstechvorrichtung (10) für die Behandlung von Akne durch Aufstechen von Pickeln, umfassend:
ein Gehäuse (15) mit einer hautkontaktierenden Oberfläche (25), wobei das Gehäuse eine Öffnung (16) in der hautkontaktierenden Oberfläche aufweist,
ein Stechelement (30), das in dem Gehäuse angeordnet ist,
ein kraftausübendes Bauelement (40), welches das Stechelement in eine Richtung treibt, um die Spitze (21) des Stechelements (30) durch die Öffnung (16) der hautkontaktierenden Oberfläche (25) des Gehäuses aus dem Gehäuse hinausragen zu lassen,
ein Auslöser-Bauelement (50) mit einem in dem Gehäuse enthaltenen Teil, der so angeordnet ist, dass er das Stechelement (30) in einer zurückgezogenen Position hält, die dem kraftausübenden Bauelement (40) Energie verleihtt, und einen zweiten Teil außerhalb des Gehäuses, der manuell betätigbar ist, um das Stechelement freizugeben, um zu bewirken, dass die Spitze eine zeitweilige, aus der Öffnung hinausragende Position einnimmt, und
**dadurch gekennzeichnet, dass** ein fluidabsorbierendes Material (60) an der Außenseite des Gehäuses (15) befestigt ist und wobei die Hautstechvorrichtung so aufgebaut ist, dass die wirksame Länge der Stechelement-Spitze (21), die aus der Hautstechvorrichtung (10) hinausragt, 100 Mikrometer bis 500 Mikrometer beträgt.

2. Hautstechvorrichtung gemäß Anspruch 1, wobei die Hautstechvorrichtung (10) ferner ein sperrendes Bauelement (70) enthält, das so aufgebaut ist, dass es im Wesentlichen verhindert, dass sich das Stechelement (30) völlig in die zurückgezogenen Position bewegt, nachdem die Spitze (21) durch die öffnung (16) hinausgetreten ist.

3. Hautstechvorrichtung gemäß einem der vorstehenden Ansprüche, wobei das kraftausübende Bauelement (40) eine Feder ist.

4. Hautstechvorrichtung gemäß einem der vorstehenden Ansprüche, wobei die Oberfläche der hautkontaktierenden Oberfläche (25) 0,5 mm² bis 25 mm² beträgt.

5. Hautstechvorrichtung gemäß einem der vorstehenden Ansprüche, wobei die hautkontaktierende Oberfläche (25) eine konkave Form aufweist und die Öffnung (16) innerhalb der konkaven Form angeordnet ist.

6. Hautstechvorrichtung gemäß einem der vorstehenden Ansprüche, wobei die Oberfläche des fluidabsorbierenden Materials (60) eine Struktur mit einem Durchmesser von 0,5 mm bis 3 mm umfasst.

7. Hautstechvorrichtung gemäß einem der vorstehenden Ansprüche, wobei das fluidabsorbierende Material (60) ein Anti-Akne-Mittel umfasst.

8. Hautstechvorrichtung gemäß Anspruch 7, wobei das Anti-Akne-Mittel ausgewählt ist aus der Gruppe bestehend aus Salicylsäure und Benzoylperoxid.

9. Hautstechvorrichtung gemäß einem der vorstehenden Ansprüche, wobei das fluidabsorbierende Material die Öffnung bedeckt und das fluidabsorbierende Material durch die Stechelement-Spitze (21) durchstochen werden kann.

10. Kit, umfassend eine Hautstechvorrichtung (10) gemäß einem der vorstehenden Ansprüche, wobei das Kit ferner wenigstens einen Gegenstand umfasst, ausgewählt aus folgender Gruppe von Gegenständen: (i) eine Zusammensetzung, die ein Anti-Akne-Mittel umfasst, (ii) ein Spannelement (36) zum Positionieren des Stechelements (30) in der zurückgezogenen Position nach der Freigabe des Stechelements, und (iii) eine Zielvorrichtung (100) zum Unterstützen der Positionierung der Hautstechvorrichtung (10) über einem zu durchstechenden Hautbereich.

11. Kit gemäß Anspruch 10, wobei das Kit ferner wenigstens eine der Zusammensetzungen umfasst, wobei das Anti-Akne-Mittel ausgewählt ist aus der Gruppe bestehend aus Salicylsäure, Benzoylperoxid, Retinolsäure, Azelainsäure, Tazoroten, Clindamycin, Adapalen, Erythromycin, Natriumsulfacetamid und Kombinationen davon.

12. Kit gemäß Anspruch 10 oder 11, wobei das Kit ferner wenigstens eines der Spannelemente (36) umfasst.

13. Kit gemäß Anspruch 10, 11 oder 12, wobei das Kit ferner wenigstens eine der Zielvorrichtungen (100) umfasst.

## Revendications

1. Dispositif de perforation de la peau (10) pour le traitement de l'acné par ouverture des boutons, comprenant :
un logement (15) comportant une surface destinée à entrer en contact avec la peau (25), ledit logement comprenant une ouverture (16) dans ladite surface destinée à entrer en contact avec la peau,
un élément de perforation (30) disposé à l'intérieur dudit logement,
un ensemble de forçage (40) pressant l'élément de perforation dans un sens pour faire dépasser la pointe (21) dudit élément de perforation (30) de l'intérieur dudit logement à travers ladite ouverture (16) dans ladite surface destinée à entrer en contact avec la peau (25) dudit logement,
un ensemble de déclenchement (50) dont une partie est contenue à l'intérieur dudit logement, disposée pour retenir ledit élément de perforation (30) dans une position rétractée mettant sous tension l'ensemble de forçage (40) et une deuxième partie est située en dehors dudit logement, actionnable manuellement pour libérer ledit élément de perforation afin d'amener momentanément ladite pointe dans une position où elle dépasse de ladite ouverture, et
**caractérisé en ce qu'**un matériau absorbeur de fluide (60) est fixé à l'extérieur dudit logement (15) et ledit dispositif de perforation de la peau étant adapté de telle sorte que la longueur efficace de la pointe de l'élément de perforation (21) qui dépasse du dispositif de perforation de la peau (10) soit de 100 micromètres à 500 micromètres.

2. Dispositif de perforation de la peau selon la revendication 1, ledit dispositif de perforation de la peau (10) comprenant en outre un ensemble restricteur (70) structuré pour empêcher sensiblement ledit élément de perforation (30) de se déplacer à fond dans ladite position rétractée après que ladite pointe (21) a dépassé de ladite ouverture (16).

3. Dispositif de perforation de la peau selon l'une quelconque des revendications précédentes, dans lequel ledit ensemble de forçage (40) est un ressort.

4. Dispositif de perforation de la peau selon l'une quelconque des revendications précédentes, dans lequel l'aire de surface de ladite surface destinée à entrer en contact avec la peau (25) est de 0,5 mm² à 25 mm².

5. Dispositif de perforation de la peau selon l'une quelconque des revendications précédentes, dans lequel ladite surface destinée à entrer en contact avec la peau (25) comprend une forme concave et ladite ouverture (16) est située à l'intérieur de ladite forme concave.

6. Dispositif de perforation de la peau selon l'une quelconque des revendications précédentes, dans lequel la surface dudit matériau absorbeur de fluide (60) comprend une structure ayant un diamètre de 0,5 mm à 3 mm.

7. Dispositif de perforation de la peau selon l'une quelconque des revendications précédentes, dans lequel ledit matériau absorbeur de fluide (60) comprend un agent anti-acné.

8. Dispositif de perforation de la peau selon la revendication 7, dans lequel ledit agent anti-acné est sélectionné dans le groupe constitué de l'acide salicylique et du peroxyde de benzoyle.

9. Dispositif de perforation de la peau selon l'une quelconque des revendications précédentes, dans lequel ledit matériau absorbeur de fluide couvre ladite ouverture et ledit matériau absorbeur de fluide est capable d'être percé par ladite pointe de l'élément de perforation (21).

10. Trousse comprenant un dispositif de perforation de la peau (10) selon l'une quelconque des revendications précédentes, ladite trousse comprenant en outre au moins un élément sélectionné dans le groupe d'éléments suivants :
(i) une composition comprenant un agent anti-acné,
(ii) un élément de réarmement (36) pour positionner ledit élément de perforation (30) dans ladite position rétractée suite à la libération dudit élément de perforation, et (iii) un dispositif de visée (100) pour aider à positionner le dispositif de perforation de la peau (10) pardessus la zone de peau à perforer.

11. Trousse selon la revendication 10, ladite trousse comprenant en outre au moins une desdites compositions, ledit agent anti-acné étant sélectionné dans le groupe constitué de l'acide salicylique, du peroxyde de benzoyle, de l'acide rétinoique, de l'acide azélaïque, du tazorotène, de la clindamycine, de l'adapalène, de l'érythromycine, du sulfacétamide sodique, et de combinaisons de ceux-ci.

12. Trousse selon la revendication 10 ou 11, ladite trousse comprenant en outre au moins un desdits éléments de réarmement (36).

13. Trousse selon la revendication 10, 11 ou 12, ladite trousse comprenant en outre au moins un desdits dispositifs de visée (100).
